# EUROPEAN PATENT SPECIFICATION

(11) **EP 2 802 223 B1**
(45) Date of publication and mention of the grant of the patent: **03.05.2017**
(21) Application number: 13703888.1
(22) Date of filing: 09.01.2013
(51) Int. Cl.: A23L 33/175, A23L 33/00, A61K 31/198, A61K 38/06

(54) **GLUTAMINE ENRICHED NUTRITIONAL COMPOSITION FOR PRETERM INFANTS**
GLUTAMINANGEREICHERTE ERNÄHRUNGSZUSAMMENSETZUNG FÜR FRÜHGEBORENE
COMPOSITION NUTRITIONNELLE ENRICHIE EN GLUTAMINE POUR NOURRISSONS PRÉMATURÉS

(30) Priority: 09.01.2012 EP 12150499; 10.01.2012 EP 12150668
(43) Date of publication of application: 19.11.2014
(73) Proprietor: N.V. Nutricia, 2712 HM Zoetermeer (NL)
(72) Inventor: VAN ELBURG, Roelof Matthijs, NL-3584 CT Utrecht (NL); BOEHM, Günther, DE-04107 Leipzig (DE)
(74) Representative: Nederlandsch Octrooibureau
(86) International application number: PCT/NL2013/050007
(87) International publication number: WO 2013/105851

(56) References cited:
- WO-A1-2011/047107
- DE KIEVIET JORRIT F ET AL: "Effects of neonatal enteral glutamine supplementation on cognitive, motor and behavioural outcomes in very preterm and/or very low birth weight children at school age", December 2012 (2012-12), BRITISH JOURNAL OF NUTRITION, VOL. 108, NR. 12, PAGE(S) 2215-2220, XP009168040, ISSN: 0007-1145(print) the whole document
- DE KIEVIET JORRIT F ET AL: "Effects of Glutamine on Brain Development in Very Preterm Children at School Age", November 2012 (2012-11), PEDIATRICS, VOL. 130, NR. 5, PAGE(S) E1121-E1127, XP009168039, ISSN: 0031-4005(print) the whole document

## Description

### FIELD OF THE INVENTION

The present invention relates to nutritional compositions for preterm and/or low birth weight infants and the long term health effects of such compositions.

### BACKGROUND OF THE INVENTION

With advances in neonatal intensive care, the survival of very preterm (born <32 weeks of gestation) and very low birth weight (VLBW; weighing <1500 grams) children has improved considerably. However, a variety of risk factors associated with preterm and low birth weight birth, including neonatal infections and inflammatory responses, adversely affect normal brain maturation processes in these children. As a consequence, widespread differences in brain development compared to term peers are found, as indicated by an overall reduction in grey and white matter volumes as measured by magnetic resonance imaging (MRI) as well as reduced white matter integrity as measured by diffusion tensor imaging (DTI). Unfortunately, these unfavourable differences in brain development appear to pertain into childhood and adolescence, and increase the risks for poor motor, cognitive, and behavioural development in very preterm/VLBW children.

Many infant formulae for term infants are available on the market. However, these are not optimal for administration to preterm infants or infants with a low birth weight, since the complete nutritional needs of a rapidly growing preterm infant are not met. Likewise, the composition of human milk does not fully meet the nutritional requirements. The major goal of enteral nutrient supply to these infants is to achieve growth similar to fetal growth coupled with satisfactory functional development. The preferred food for infants with a low birth weight is therefore fortified human milk, or, alternatively, formula specially designed for these infants. Human milk fortifiers (HMF) and preterm infant formulae are commercially available. Typically, HMF comprises besides protein, also carbohydrates or fat, minerals and vitamins. Typically, preterm infant formulae have an increased protein concentration. Enteral feeding of very low birth weight (VLBW) infants is a challenge, since metabolic demands are high and administration of enteral nutrition is limited by immaturity of the gastrointestinal tract.

The amino acid glutamine has been used in nutritional compositions for preterm infants. Van den Berg et al, 2005, Am J Clin Nutr 81:1397-1404 disclose that supplementation with glutamine lowers infectious morbidity in VLBL infants. Van den Berg et al, 2007, Arch. Pediatrics & Adolescent Medicine, 161:1095-1101 disclose that supplementation with glutamine decreases risk for atopic dermatitis in the first year of life and at the age of 6. Van Zwol et al, 2008, Acta Paediatrica, 97:562-567 report that there is no effect of glutamine supplementation in VLBW infants on neurodevelopment outcome at age of 2 years. WO 2011/047107 discloses the use of an arginine-glutamine dipeptide to support retinal, intestinal and/or nervous system development in a neonate.
Effects on structural brain development and long term functional brain development have not been reported so far.

### SUMMARY OF THE INVENTION

The inventors found that glutamine enriched feeding in the first month after birth in very preterm and/or VLBW infants has a long term effect on measures of brain development. In total 52 very preterm and/or VLBW children, who originally took part in a randomized controlled trial on enteral glutamine supplementation between day 3 and 30 of life, participated in this follow up study at school age, with a mean age of 8.6 years (SD = 0.3 years). Measures of brain development included volumetric outcomes of various brain structures as well as fractional anisotropy (FA) values of major white matter tracts, using Magnetic Resonance Imaging (MRI) including Diffusion Tensor Imaging (DTI) techniques, respectively.

In term infants followed up at school age an increased cortical and subcortical volume was observed compared to the preterm born group. Advantageously an increased cortical brain volume and subcortical brain volume was also observed in children who were born preterm and were supplemented in the first month after birth with L-glutamine when compared to the control group of preterm infants not having received L-glutamine. In particular a significant increased white matter volume, brain stem volume, and hippocampus volume was observed

### DETAILED DESCRIPTION OF THE INVENTION

The present invention thus concerns a glutamine enriched nutritional composition for use as disclosed in present claim 1.

Preferably the glutamine enriched nutritional composition is administered during the first six, preferably the first four, more preferably the first two and most preferably at least the first month after birth of the low birth weight and/or preterm infant.

### Preterm and LBW infants

The present invention relates to a method for feeding an infant selected from the group consisting of low birth weight infants (LBW infants), very low birth weight infant (VLBW infants), extremely low birth weight infants (ELBW), preterm (=premature) infants and very premature infants. LBW infants have a weight of less than 2500 grams at birth. VLBW infants have a weight below 1500 grams at birth. ELBW infants have a birth weight below 1000 grams at birth. Preterm infants are born before the end of the 37th week of pregnancy. Very preterm infants are born before the end of the 32th week of pregnancy. Preferably the infants are selected from the group of ELBW, VLBW, and very preterm infants.

### Glutamine

The nutritional composition of the present invention comprises glutamine. Throughout this description this is also referred to as nutritional composition comprising glutamine or glutamine enriched nutritional composition. Glutamine in the present invention refers to L-glutamine. Glutamine is one of the most abundant amino acids in plasma and human milk and is considered conditionally essential in preterm infants. Glutamine is utilized as a source of energy and for nucleotide synthesis in all rapidly dividing cells, such as the intestinal lining and certain immune cells. In the brain, glutamine is a substrate for neurotransmitters and an important source of energy for the nervous system. LBW or preterm, in particular VLBW and/or very preterm infants, may be especially susceptible to glutamine depletion as nutritional supply of glutamine is limited in the first weeks after birth. Furthermore the endogenous capacity to synthesize glutamine from glutamate is not fully developed in these infants.

Glutamine is preferably present in an easily absorbable form. Because of the immaturity of the intestinal tract of the LBW and/or preterm infant, glutamine present in intact protein is less easily absorbed. Therefore the glutamine is preferably present in the form of free amino acids and/or di- and/or tripeptides, most preferably glutamine dipeptide and/or free glutamine. Free glutamine and glutamine dipeptide are commercially available, for example at Ajinomoto, USA. In one embodiment glutamine dipeptide is in the form of a dipeptide of 2 glutamine residues. In one embodiment, glutamine dipeptide is in the form of a dipeptide of one glutamine and one amino acid other than glutamine. For example glutamine dipeptide can also be in the form of alanine-glutamine dipeptide. The term free glutamine in the context of this invnetion means in the form of a single amino acid. This includes glutamine as a single amino acid in the form of a salt.

The nutritional composition of the present invention comprises glutamine levels higher then normally present in human milk protein or standard preterm formula based on cow's milk derived protein. Preferably the nutritional composition of the present invention comprises at least 12 wt.%, more preferably at least 15 wt.%, more preferably at least 20 wt.%, even more preferably at least 30 wt.% glutamine based on total protein. Preferably the nutritional composition of the present invention comprises at least 1.5 wt.%, more preferably at least 2 wt.%, even more preferably at least 4 wt.% glutamine based on dry weight of the nutritional composition. Preferably the nutritional composition of the present invention comprises at least 0.3 g, more preferably at least 0.5 g, even more preferably at least 1 g glutamine based on 100 kcal. Preferably the nutritional composition of the present invention comprises at least 12 wt.%, more preferably at least 15 wt.%, more preferably at least 20 wt.%, even more preferably at least 30 wt.% glutamine in the form of free amino acid and/or dipeptide based on total protein. Preferably the nutritional composition of the present invention comprises at least 1.5 wt.%, more preferably at least 2 wt.%, even more preferably at least 4 wt.% glutamine in the form of free amino acid and/or dipeptide based on dry weight of the nutritional composition. Preferably the nutritional composition of the present invention comprises at least 0.3 g, more preferably at least 0.5 g, even more preferably at least 1 g glutamine in the form of free amino acid and/or dipeptide based on 100 kcal.

The daily dose of glutamine, preferably free glutamine, based on kg body weight, is preferably of 0.01 to 0.5 g, more preferably 0.03 to 0.4 g, even more preferably 0.07 to 0.35 g.

### Nutritional composition

In one embodiment the glutamine enriched composition of the present invention is a nutritional composition. In one embodiment, the present invention relates to nutritional compositions for use in feeding LBW and/or preterm infants, more preferably VLBW and/or very preterm infants.

In one embodiment the glutamine enriched nutritional composition of the present invention is a preterm formula. The preterm formula comprises all macro- and micronutrients needed for preterm infants so as to achieve a growth similar to fetal growth coupled with satisfactory functional development.

In one embodiment, the invention concerns a nutritional composition comprising between 1.5 wt.% and 20 wt.% glutamine based on dry weight of the nutritional composition, and comprising protein in such an amount that glutamine is present between 12 wt.% and 80 wt% based on total protein. Preferably the nutritional composition is a preterm formula. Preferably the glutamine is in the form of free glutamine, glutamine dipeptide and/or glutamine tripeptide.

In a preferred embodiment the preterm formula comprises from 5 to 25 wt.% protein, preferably 9 to 20 wt.%, more preferably 13 to 18 wt.% protein based on the dry weight of the preterm formula. In a preferred embodiment the preterm formula comprises from 1.8 to 3.0 g protein, preferably, preferably 2.0 to 3.0 g, preferably 2.5 g to 2.6 g protein, per 100 ml.

In a preferred embodiment the preterm formula comprises from 6 to 30 % protein, more preferably 10 to 20, even more preferably 12 to 15 % protein, based on total calories. The wording "% protein based on total calories" refers to the energy of 1 g protein being 4.0 kcal. For determining the amount of protein, the sum of proteins, peptides and free amino acids, including the glutamine, should be taken into account. Preferably, the protein content is determined according to the Kjeldahl method using a conversion factor of 6.25 and preferably using a conversion factor of 6.38 in case of casein dominant formula.

Whey protein is highly suitable as a protein source for preterm infants with a low body weight. However, since sweet whey protein generally has a high threonine content, which can result in hyperthreoninemia, preferably the present preterm formula, comprises acid whey protein or sweet whey protein from which at least part of the glycomacropeptide (GMP) is removed. The present preterm formula, preferably contains from 4.7 to 7.5 g threonine per 100 g amino acids, preferably from 3.9 to 4.6 g threonine per 100 g amino acids. Preferably, the present preterm formula, contains non-hydrolysed protein, in order to decrease the osmolarity of the reconstituted preterm formula, which is beneficial in LBW and/or preterm infants, especially in VLBW and ELBW and/or very preterm infants. Advantageously, the protein of the present preterm formula is hydrolysed in order to increase digestibility and gastro-intestinal tolerance. This is particularly preferred in LBW and/or preterm infants and in particular in VLBW and ELBW and/or very preterm infants since they have an immature intestinal tract which impaired digestion and absorption capacity. Advantageously and preferably, the protein is present in the preterm formula in partially hydrolyzed form, thereby enabling a better solubility and digestion than intact protein, without increasing the osmolarity too much, as is the case with extensively hydrolysed protein or free amino acids. Partially hydrolyzed protein in this invention refers to protein with a degree of hydrolysis from 3 to 20 %. In a preferred embodiment of the invention the preterm formula comprises a protein with a degree of hydrolysis from 3 to 20 %, preferably 5 to 18 %, most preferably 5 to 15 %.

In a preferred embodiment of the invention the preterm formula comprises casein and/or whey protein. In a preferred embodiment of the invention the preterm formula comprises casein. In a preferred embodiment of the invention the preterm formula comprises whey protein. Preferably, the preterm formula comprises whey protein and casein derived from non-human milk, preferably cow's milk. Preferably, the weight ratio (based on dry weight) of casein to whey protein is from 80/20 to 20/80, more preferably from 40/60 to 50/50.

Preferably the preterm formula of the present invention comprises at least 12 wt.%, more preferably at least 15 wt.%, more preferably at least 20 wt.%, even more preferably at least 30 wt.% glutamine based on total protein. Preferably the preterm formula of the present invention comprises no more than 80 wt.%, more preferably no more than 50 wt.% glutamine based on total protein. Preferably the preterm formula of the present invention comprises at least 1.5 wt.%, more preferably at least 2 wt.%, even more preferably at least 4 wt.% glutamine based on dry weight of the preterm formula. Preferably the preterm formula of the present invention comprises no more than 20 wt.%, more preferably no more than 10 wt.% glutamine based on dry weight of the preterm formula. Preferably the nutritional composition of the present invention comprises at least 0.3 g, more preferably at least 0.5 g, even more preferably at least 1 g glutamine based on 100 kcal of the preterm formula. Preferably the preterm formula of the present invention comprises no more than 5 g, even more preferably no more than 2 g glutamine based on 100 kcal of the preterm formula.

Preferably the preterm formula of the present invention comprises at least 12 wt.%, more preferably at least 15 wt.%, more preferably at least 20 wt.%, even more preferably at least 30 wt.% glutamine in the form of free amino acid and/or dipeptide based on total protein. Preferably the preterm formula of the present invention comprises no more than 80 wt.%, more preferably no more than 50 wt.% glutamine in the form of free amino acid and/or dipeptide based on total protein. Preferably the preterm formula of the present invention comprises at least 1.5 wt.%, more preferably at least 2 wt.%, even more preferably at least 4 wt.% glutamine in the form of free amino acid and/or dipeptide based on dry weight of the preterm formula. Preferably the preterm formula of the present invention comprises no more than 20 wt.%, more preferably no more than 10 wt% glutamine in the form of free amino acid and/or dipeptide based on dry weight of the preterm formula. Preferably the nutritional composition of the present invention comprises at least 0.3 g, more preferably at least 0.5 g, even more preferably at least 1 g glutamine in the form of free amino acid and/or dipeptide based on 100 kcal of the preterm formula. Preferably the preterm formula of the present invention comprises no more than 5 g, even more preferably no more than 2 g glutamine in the form of free amino acid and/or dipeptide based on 100 kcal of the preterm formula.

The preterm formula of the present invention in ready to drink form has in a preferred embodiment about 70 to 90 kcal, preferably 75 to 85 kcal per 100 ml.

Preferably the preterm formula has an osmolarity below 450 mOsmol/l, more preferably below 400, even more preferably below 350. Particularly in preterm infants, a too high osmloartiy is a disadvantage.

Preferably, the preterm formula comprises about 3.5 to 5.5 g, preferably 3.9 to 5.0 g, fat per 100 ml. Preferably the preterm formula comprises about 20 to 50 wt.%, preferably 26 to 35 wt.% fat based on dry weight of the preterm formula. Preferably the preterm formula comprises about 3.5 to 7.0 g, more preferably 5.0 to 6.5 g, more preferably 5.3 to 5.8 g fat, based on 100 kcal of the preterm formula. Fat is preferably vegetable fat. Preferably the fat component comprises long chain poly-unsaturated fatty acids such as docosahexaenoic acid and arachidonic acid. These fatty acids are beneficial for brain and visual development in preterm and/or LBW infants. Preferably the fat component comprises medium chain fatty acids, preferably at least 10 wt% and at most 40 wt% based on total fatty acids. Medium chain fatty acids are more easily absorbed by preterm and/or LBW infants.

Preferably, the preterm formula comprises 6 to 10 g, preferably 7 to 8 g, digestible carbohydrates per 100 ml. Preferably the preterm formula comprises 40 to 80 wt.% preferably 40 to 50 wt.% digestible carbohydrates based on dry weight of the preterm formula. Preferably the preterm formula comprises about 5 to 20 g, more preferably 6 to 15 g, more preferably 7 to 10 g digestible carbohydrates, based on 100 kcal of the preterm formula. Suitable digestible carbohydrates are lactose and maltodextin.

Preferably, the preterm formula according to the present invention comprises at least one non-digestible oligosaccharide. Advantageously and most preferred, the non-digestible oligosaccharide is water-soluble (according to the method disclosed in L. Prosky et al, J. Assoc. Anal. Chem 71: 1017-1023, 1988). Non-digestible oligosaccharides, also called prebiotics, are not digested in the intestine by the action of digestive enzymes present in the human upper digestive tract (small intestine and stomach) but instead are fermented by the human intestinal microbiota. For example, glucose, fructose, galactose, sucrose, lactose, maltose and the maltodextrins are considered digestible. Non-digestible oligosaccharides advantageously further improve the effect of the present glutamine on structural brain volume or growth. Non-digestible oligosaccharides stimulate a healthy gut environment, thereby aiding the uptake of glutamine.

Preferably the non-digestible oligosaccharide has a degree of polymerisation (DP) of 2 to 200. The average DP of the non-digestible oligosaccharide is preferably below 200, more preferably below 100, even more preferably below 60, most preferably below 40. The non-digestible oligosaccharide is preferably selected from the group consisting of fructo-oligosaccharide, such as inulin, non-digestible dextrin, galacto-oligosaccharide, such as transgalacto-oligosaccharide, xylo-oligosaccharide, arabino-oligosaccharide, arabinogalacto-oligosaccharide, gluco-oligosaccharide, such as gentio-oligosaccharide and cyclodextrin, glucomanno-oligosaccharide, galactomanno-oligosaccharide, mannan-oligosaccharide, chito-oligosaccharide, sialyloligosaccharide, such as 3-SL, 6-SL, LSTa,b,c, DSLNT, S-LNH, DS-LNH, and fuco-oligosaccharide, such as (un)sulphated fucoidan OS, 2-FL, 3-FL, LNFP I, II, III, V, LNnFPI, LNDH, and mixtures thereof, more preferably fructo-oligosaccharide, such as inulin, galacto-oligosaccharide, such as transgalacto-oligosaccharide, which is β-linked, and fuco-oligosaccharide and mixtures thereof, even more preferably transgalacto-oligosaccharide. When the non-digestible oligosaccharide is a mixture, the averages of the respective parameters are used for defining the present invention.

The present invention preferably provides a composition with two different non-digestible oligosaccharides, with a different type of glycosidic linkage, a different degree of polymerisation and/or a different monosaccharide composition. Here below the two different non-digestible oligosaccharides are also referred to as non-digestible oligosaccharide A and B.

Preferably, at least 60%, more preferably at least 75% even more preferably 90%, most preferably 98% of the total monosaccharide units of the non-digestible oligosaccharide, are monosaccharides selected from the group consisting of galactose (gal), fructose (fru) and glucose (glu) monosaccharides.

Preferably the non-digestible oligosaccharide is an oligosaccharide selected from the group consisting of β-galacto-oligosaccharide, α-galacto-oligosaccharide, and galactan. According to a more preferred embodiment non-digestible oligosaccharide A is β-galacto-oligosaccharide. β-galacto-oligosaccharide is also sometimes referred to as transgalacto-oligosaccharide. Preferably non-digestible oligosaccharide A comprises galacto-oligosaccharides with β(1,4), β(1,3) and/or β(1,6) glycosidic bonds and a terminal glucose. Transgalacto-oligosaccharide is for example available under the trade name Vivinal®GOS (Borculo Domo Ingredients, Zwolle, Netherlands), Bi2muno (Clasado), Cup-oligo (Nissin Sugar) and Oligomate55 (Yakult).

Preferably non-digestible oligosaccharide is a fructo-oligosaccharide. A fructo-oligosaccharide may in other context have names like fructopolysaccharides, oligofructose, polyfructose, polyfructan, inulin, levan and fructan and may refer to oligosaccharides comprising β-linked fructose units, which are preferably linked by β(2,1) and/or β(2,6) glycosidic linkages, and a preferable DP between 2 and 200. Preferably, the fructo-oligosaccharide contains a terminal β(2,1) glycosidic linked glucose. Preferably, the fructo-oligosaccharide contains at least 7 β-linked fructose units. In a further preferred embodiment inulin is used as non-digestible oligosaccharide B. Inulin is a type of fructo-oligosaccharide wherein at least 75% of the glycosidic linkages are β(2,1) linkages. Typically, inulin has an average chain length between 8 and 60 monosaccharide units. A suitable fructo-oligosaccharide for use in the compositions of the present invention is commercially available under the trade name Raftiline®HP (Orafti). Other suitable sources are raftilose (Orafti), fibrulose and fibruline (Cosucra) and Frutafit and frutalose (Sensus).

Most preferred is a mixture of transgalacto-oligosaccharide with an average DP below 10, preferably below 6 and a fructo-oligosaccharide with an average DP above 7, preferably above 11, even more preferably above 20.

If the present preterm formula comprises galacto- and fructo-oligosaccharides the weight ratio is preferably from 1/99 to 99/1, more preferably from 1/19 to 19/1, even more preferably from 1 to 19/1.

Preferably, the present preterm formula comprises the non-digestible oligosaccharides transgalacto-oligosaccharide and fructo-oligosaccharide.

Preferably, the present invention relates to a preterm formula, wherein the non-digestible oligosaccharide is selected from the group consisting of fructo-oligosaccharide and galacto-oligosaccharide.
The present nutritional composition preferably comprises 0.05 to 20 wt.% total non-digestible oligosaccharide, more preferably 0.5 to 15 wt.%, even more preferably 1 to 10 wt.%, most preferably 2.0 to 10 wt.%, based on dry weight of the present composition. Based on 100 ml the present nutritional composition preferably comprises 0.01 to 2.5 wt.% total non-digestible oligosaccharide, more preferably 0.05 to 1.5 wt.%, even more preferably 0.25 to 1.5 wt.%, based on 100 ml of the present composition.

In one embodiment the present invention concerns a glutamine-based supplement, suitable to fortify human milk, to fortify human milk fortified with a standard human milk fortifier or to fortify a standard preterm formula. In the context of this invention, a supplement does not comprise all macro- and micronutrients needed for preterm infants so as to achieve a growth similar to fetal growth coupled with satisfactory functional development.

Standard preterm formula, and human milk fortified with standard human milk fortifier comprises all macro- and micronutrients needed for preterm infants so as to achieve a growth similar to fetal growth coupled with satisfactory functional development. In standard preterm formula, standard human milk fortifier, human milk and human milk fortified with standard human milk fortifier the amount of glutamine, more preferably free glutamine or glutamine in the form of a dipeptide, is below 12 wt.% based on total protein, also the amount of glutamine is below 1.5 wt.% based on dry weight, and also the amount of glutamine is below 0.3 g per 100 kcal. The composition of a standard preterm formula is similar as described above, except for the glutamine amounts, which are lower.

The glutamine based supplement according to the present invention is preferably packed in unit dose form. Preferably, the glutamine based supplement is present in a container. The container may contain more than one unit dose, preferably the container contains one single unit dose. Preferably the container is a sachet. Also preferably the container is a capsule or an ampoule. In a preferred embodiment of the invention the glutamine-based supplement is present in a container, in unit dose form, and in dry form, preferably in the form of a powder, and is present in an amount of from 0.05 to 10 g, preferably 0.05 g to about 8 g powder, preferably 0.1 to 4 g even more preferable 0.2 to 1 g powder per container. Preferably the container is provided with instructions for adding at least a portion of said glutamine-based supplement to human milk, to standard preterm formula, or to human milk fortified with standard HMF. Preferably, the nutritional supplement is a reconstitutable powder. Powdered nutritional supplements have the advantage that they minimize the volume displacement of preterm formula or human milk.

In one embodiment, the invention concerns a glutamine-based supplement in the form of a powder in a unit dose comprising at least 15 wt.% glutamine in the form of free glutamine, glutamine dipeptide and/or glutamine tripeptide on dry weight of the powder.

Preferably the glutamine-based supplement of the present invention comprises at least 15 wt.%, more preferably at least 20 wt.%, even more preferably at least 30 wt.% glutamine based on total protein, even more preferably more than 50 wt.%, most preferably more than 75 wt.% glutamine based on total protein. Preferably the glutamine-based supplement of the present invention comprises at least 1.5 wt.%, more preferably at least 2 wt.%, more preferably at least 4 wt.% glutamine, even more preferably at least 15 wt.%, even more preferably at least 40 wt.%, most preferably at least 75 wt.% glutamine based on dry weight of the supplement. Preferably the glutamine-based supplement of the present invention comprises at least 0.3 g, more preferably at least 0.5 g, more preferably at least 1 g glutamine, even more preferably at least 5, more preferably at least 10, most preferably at least 20 g glutamine based on 100 kcal.

Preferably the glutamine-based supplement of the present invention comprises at least 15 wt.%, more preferably at least 20 wt.%, even more preferably at least 30 wt.% glutamine in the form of free amino acid and/or dipeptide based on total protein, even more preferably more than 50 wt.%, most preferably more than 75 wt.% glutamine in the form of free amino acid and/or dipeptide based on total protein. Preferably the glutamine-based supplement of the present invention comprises at least 1.5 wt.%, more preferably at least 2 wt.%, more preferably at least 4 wt.%, even more preferably at least 15 wt.%, even more preferably at least 40 wt.%, most preferably at least 75 wt.% glutamine in the form of free amino acid and/or dipeptide based on dry weight of the supplement. Preferably the glutamine-based supplement of the present invention comprises at least 0.3 g, more preferably at least 0.5 g, more preferably at least 1 g glutamine, even more preferably at least 5, more preferably at least 10, most preferably at least 20 g glutamine in the form of free amino acids and/or dipeptide based on 100 kcal.

In one embodiment, the glutamine-based supplement comprises further components such as proteins other than free glutamine, carbohydrates, fats, minerals like calcium and-or vitamins. In one embodiment, the glutamine-based supplement comprises non digestible oligosaccharides as discussed above.

Preferably the glutamine-based supplement is added to human milk, standard preterm formula, or human milk fortified with standard human milk fortifier in such an amount that the final glutamine enriched nutritional composition comprises at least 12 wt.%, more preferably at least 15 wt.%, more preferably at least 20 wt.%, even more preferably at least 30 wt.% glutamine based on total protein. Preferably the nutritional supplement is added to human milk, standard preterm formula, or human milk fortified with standard human milk fortifier in such an amount that the final glutamine enriched nutritional composition comprises at least 1.5 wt.%, more preferably at least 2 wt.%, even more preferably at least 4 wt.% glutamine based on dry weight of the nutritional composition. Preferably the nutritional supplement is added to human milk, standard preterm formula, or human milk fortified with standard human milk fortifier in such an amount that the final glutamine enriched nutritional composition comprises at least 0.3 g, more preferably at least 0.5 g, even more preferably at least 1 g glutamine based on 100 kcal.

Preferably the nutritional supplement is added to human milk, standard preterm formula, or human milk fortified with standard human milk fortifier in such an amount that the final glutamine enriched nutritional composition comprises at least 12 wt.%, more preferably at least 15 wt.%, more preferably at least 20 wt.%, even more preferably at least 30 wt.% glutamine in the form of free amino acid and/or dipeptide based on total protein. Preferably the nutritional supplement is added to human milk, standard preterm formula, or human milk fortified with standard human milk fortifier in such an amount that the final glutamine enriched nutritional composition comprises at least 1.5 wt.%, more preferably at least 2 wt.%, even more preferably at least 4 wt.% glutamine in the form of amino acid and/or dipeptide based on dry weight of the nutritional composition. Preferably the nutritional supplement is added to human milk, standard preterm formula, or human milk fortified with standard human milk fortifier in such an amount that the final glutamine enriched nutritional composition comprises at least 0.3 g, more preferably at least 0.5 g, even more preferably at least 1 g glutamine in the form of free amino acid and/or dipeptide based on 100 kcal.

Preferably the glutamine-based supplement comprises at least 10 wt.% non digestible oligosaccharides based on dry weight of the glutamine-based supplement, preferably at least 15 wt.%, more preferably at least 20 wt.%, more preferably at least 25 wt.%, or at least 30 wt.% or at least 40 wt.% up to at most 50 wt.% non digestible oligosaccharides based on dry weight of the glutamine-based supplement.

Preferably, the non digestible oligosaccharides in the glutamine-based supplement invention are selected from the group consisting of fructo-oligosaccharide and galacto-oligosaccharide.

Preferably, the glutamine-based supplement comprises the non-digestible oligosaccharides transgalacto-oligosaccharide and fructo-oligosaccharide.

Preferably the present nutritional composition comprising glutamine is in dry form, preferably a powder. This powder is suitable for reconstitution with water or another aqueous phase. When glutamine is in powder form it advantageously has a better shelf life. Glutamine, in particular free glutamine and glutamine dipeptide, is more stable when stored in dry form.

In one embodiment, the present invention concerns a method for preparing a glutamine-enriched nutritional composition comprising adding glutamine to a nutritional composition. Preferably the glutamine that is added is in the form of glutamine free amino acid, glutamine dipeptide, and/or glutamine tripeptide, preferably in the form of free glutamine and/or glutamine dipeptide. Preferably the nutritional composition to which glutamine is added is human milk, standard preterm formula or human milk fortified with standard human milk fortifier.

### Application

Administration of the present nutritional composition to the preterm and/or LBW infant is enterally or parenterally, more preferably enterally, more preferably orally via a bottle, even more preferably enterally by naso-gastric tube feeding. Enteral feeding has the advantage that the glutamine will provide a beneficial effect in the intestinal tract as well regarding intestinal infections and improving barrier function.

Administration of the nutritional composition comprising glutamine is preferably during the first month after birth, more preferably until a body weight of 2500 is reached. Preferably the administration is daily. Preferably the administration is daily for at least a period of a week, more preferably 30 days.

The inventors found that glutamine enriched feeding in the first month after birth in very preterm and/or VLBW infants has a long term effect on measures of brain development. Advantageously an increased cortical brain volume and subcortical brain volume was also observed in children who were born preterm and were supplemented in the first month after birth with glutamine when compared to the control group of preterm infants not having received glutamine. In particular a statistically significant increased white matter volume, brain stem volume, and hippocampus volume was observed. Disclosed herein is a nutritional composition comprising glutamine for use in increasing brain volume and/or brain mass in preterm and/or LBW infants. With increase brain volume and/or brain mass is meant an increase to a value more similar to a term infant control group. Alternatively, with increase brain volume and/or brain mass is meant an increase compared to a preterm control group not having received the nutritional composition comprising glutamine of the present invention. Alternatively,with increase of brain mass and/or brain volume also increase in brain size or brain tissue is meant.
In particular an increase in brain volume and/or mass refers to an increase of volume and/or mass in brain structure selected from the group consisting of cortical brain, intracranial volume, grey matter, white matter and cerebellum, subcortical brain, brain stem, thalamus, putamen, caudate nucleus, hippocampus, globus pallidum, amygdale, nucleus accumbens and the corpus callosum, more particular white matter, brain stem, and hippocampus. Disclosed herein is a nutritional composition comprising glutamine for use in improving growth of brain volume and/or mass in preterm and/or LBW infants. With improving growth of brain volume and/or brain mass is meant an increased growth to a value more similar to a term infant control group, thus preferably an increased growth compared to the growth in a term control group to arrive at a value of brain volume and/or brain mass more similar to that in the term control group. Alternatively, with improving growth of brain volume and/or mass in preterm and/or LBW infants is meant an increased growth compared to a preterm control group not having received the nutritional composition comprising glutamine of the present invention. With improving growth of brain mass and/or brain volume also improving of structural brain development is meant. With improving growth of brain mass and/or brain volume also stimulating growth of brain mass and/or brain volume and stimulating of structural brain development is meant. With improving growth of brain mass and/or brain volume also increased growth of brain mass and/or brain volume and increased structural brain development is meant.

In particular an improvement of growth of brain volume and/or mass refers to an improvement of growth of volume and/or mass of brain structure selected from the group consisting of cortical brain, intracranial volume, grey matter, white matter and cerebellum, subcortical brain, brain stem, thalamus, putamen, caudate nucleus, hippocampus, globus pallidum, amygdale, nucleus accumbens and the corpus callosum, more particular white matter, brain stem, and hippocampus.

Smaller total brain volumes, white and grey matter volumes, as well as smaller volumes of the cerebellum, hippocampus, and corpus callosum have been found associated with lower outcomes in terms of IQ by at least one study, see Allin et al., Brain 2001, 1: 60-6; Taylor et al., Dev Neuropsychol 2011, 1: 96-117; Parker et al., Brain 2008, 5: 1344-51; Allin et al., Arch Pediatr Adolesc Med 2007, 12: 1183-9; Martinussen et al., J Pediatr 2009, 6: 848-53; Narberhaus et al., Neuropsychologia 2008, 1: 111-6; Reiss et al., J Pediatr 2004, 2:242-9; Yung et al., Pediatr Res 2007, 6: 732-6; Northam et al., Ann Neurol 2011, 4: 702-11. The nutritional composition comprising glutamine according to the present invention is for improving neurocognitive functioning, later in life in infants born preterm and/or with low birth weight.

The effects on the brain structure and/or functioning as described above are preferably established later in life. The effects on the brain as described above are preferably established during school age. With school age an age between 4 and 12 years is meant. It is particularly relevant that the observed effects of the nutritional composition comprising glutamine on the brain are established during school age, since then the neurocognitive function is more evident.

Preferably the nutritional composition comprises between 1.5 wt.% and 20 wt.% glutamine based on dry weight of the nutritional composition, and comprising protein in such an amount that glutamine is present between 12 wt.% and 80 wt% based on total protein. Preferably the nutritional composition is a preterm formula. Preferably the glutamine in the nutritional composition is in the form of free glutamine, glutamine dipeptide and/or glutamine tripeptide, preferably free glutamine and/or glutamine dipeptide. Preferably the nutritional composition or glutamine-based supplement further comprises non-digestible oligosaccharides selected from the group consisting of galacto-oligosaccharides and fructo-oligosaccharides, preferably in an amount as defined above. Preferably the nutrition to a low birth weight and/or preterm infant is provided in the first month after birth, preferably during the first month after birth. Preferably the nutritional composition according to the present invention, or a glutamine-based supplement according to the present invention, is for use in providing nutrition to a low birth weight and/or preterm infant, for use in improving neurocognitive functioning, at an age from 4 to 12 years.

In one embodiment the low birth weight or preterm infant is a very low birth weight infant or a very premature infant. In one embodiment, the daily dose of glutamine provided to the infant is at least 0.01 to 0.5 g/kg body weight, preferably 0.03 to 0.4, even more preferably 0.07 to 0.35 g/kg body weight. In one embodiment the nutritional composition comprises galacto-oligosaccharides. In one embodiment, the nutritional composition is a glutamine-based supplement in the form of a powder in a unit dose comprising at least 15 wt.% glutamine in the form of free glutamine, glutamine dipeptide and/or glutamine tripeptide on dry weight of the powder. In one embodiment, the glutamine-based supplement comprises at least 10 wt.% non digestible oligosaccharides based on dry weight of the glutamine-based supplement.

### EXAMPLES

### Example 1: Effect of glutamine supplementation in VLBW and/or very preterm infants on brain volume later in life

A randomized, placebo controlled clinical trial was performed. For a detailed protocol of the study, see van den Berg et al, 2004, Glutamine-enriched enteral nutrition in very low birth weight infants. Design of a double-blind randomised controlled trial [ISRCTN73254583], BMC Pediatrics 4:17.

In short: Infants with a gestational age <32 weeks and/or birth weight <1500 g were enrolled. In total 52 very preterm and/or VLBW children, which had taken part in a randomized controlled trial on enteral glutamine supplementation between day 3 and 30 of life, participated in this follow up study at the mean age of 8.6 years (SD = 0.3 years). 30 infants were in the control group and 22 in the glutamine supplementation group. Also comparisons were made with a control group (n=52) of term born infants.

The glutamine nutritional supplement was in powder form and contained 82 wt.% L-glutamine in total and 18 wt.% glucose (nitrogen 15.5 wt/wt%; 371 kcal/100 g), whereas the isonitrogenous control powder contained 100 wt.% L-alanine (nitrogen 15.7 wt/wt%, 435 kcal/100 g). The two powders were indistinguishable by appearance, colour and smell. Between days 3 and 30 of life, supplementation was administered in increasing doses to a maximum of 0.3 g/kg glutamine per day in the glutamine group. Initially, the supplementation dose was based on birth weight. After 2 weeks the dose was adjusted to actual body weight. Two members of the nursing staff daily added supplementation to breast milk or to preterm formula (Nenatal®, Nutricia Nederland B.V., Zoetermeer, The Netherlands), according to the parents' choice. Per 100 ml, Nenatal® provides 78 kcal, 2.1 g protein (casein-whey protein weight ratio 40:60), 4.4 g fat and 7.5 g carbohydrate. Nenatal® does not contain free glutamine and alanine or glutamine and alanine in the form of dipeptide. Protocol guidelines for the introduction of parenteral and enteral nutrition are disclosed in van den Berg et al., 2004.

All statistical analyses are performed on an intention to treat basis. In addition, alternative per protocol analyses are performed, excluding all patients who are not treated according to protocol, defined as more than 3 consecutive days or more than a total of 5 days on minimal enteral feeding or without supplementation. A p value <0.05 is considered significant (two-tailed). SPSS 9.0 (SPSS Inc., Chicago, IL, USA) and STAT 7.0 (Stat-Corp LP, College Station, TX, USA) are used for data analysis. A p value of 0.1 or below is considered to represent a realistic effect, a trend. Due to the small size of the groups also the effect size is taken into account. An effect size above 0.4 is considered to represent a trend.

### Results:

No statistically significant difference of the control preterm group and glutamine supplemented preterm group for follow up was present regarding base line characteristics, such as age, social economic status, ethnicity, birth weight, gestational age, head circumference, HELLP syndrome, BPD (bronchopulmonary dysplasia), IVH (intraventricular hemorrhage) grade I/II, IVH grade III/IV, ROP (retinopathy of prematurity) grade III/IV, Apgar score after 5 min., mode of delivery, gender and drop outs. The control group had more infections and a higher use of prenatal corticosteroids. Mean birth weight was 1186 (sd 336) gram in the control group, and 1252 (sd 380) in the glutamine group. Gestational age was 28.9 (sd 1.7) weeks in the control group and 29.4 (sd 1.7) in the glutamine group.

Brain cortical volumes were determined by MRI and calculated using FSL software "FAST" and corrected for age at MRI scan, gender and body weight for gestational age.
Brain cortical volumes were significantly higher in the term control group than in the preterm group. This was the case for intracranial volume, grey matter, white matter and cerebellum volume. In the preterm group, the glutamine supplemented group had a significantly higher white matter volume. A tendency for an increase in intracranial volume, and cerebellum volume was also observed, see Table 1. The cortical brain volumes in the glutamine group were higher than in the control group, and were more comparable to the term control group.

**Table 1: Cortical volumes in children born preterm and being supplemented with L-glutamine or with control during the first month of life.**

| Brain structures (in cm³) | Control | Glutamine | Term infants | Effect size* | P-value* |
|---|---|---|---|---|---|
| Intracranial volume (sd) | 1574.1 (134.4) | 1649.8 (179.3) | 1654.1 (132.7) | 0.48 | 0.07 |
| Grey matter (sd) | 705.4 (63.3) | 727.4 (85.60) | 735.8 (53.6) | 0.29 | 0.39 |
| White matter (sd) | 466.5 (49.2) | 496.5 (60.4) | 494.5 (49.6) | 0.54 | 0.03 |
| Cerebellum (sd) | 105.7 (9.5) | 110.89 (15.3) | 113.2 (11.1) | 0.41 | 0.13 |

| | | | | | |
|---|---|---|---|---|---|
| * For control versus glutamine group | | | | | |

Brain subcortical volumes were determined by MRI and calculated using FSL software "FAST" and corrected for age at MRI scan, gender and body weight for gestational age.

Brain subcortical volumes were significantly higher in the term controls than in preterm group. This was the case for the brain stem, thalamus, putamen, caudate nucleus, hippocampus, globus pallidum, amygdale, nucleus accumbens and the corpus callosum volume. In the preterm group, the glutamine supplemented group had higher subcortical volume, and significantly higher brain stem volume and hippocampus volume, and a tendency for an increase in other subcortical brain structures volume, see Table 2. The subcortical brain volumes in the glutamine group were higher than in the control group, and were more comparable to the term control group.

**Table 2: Subcortical volumes in children born preterm and being supplemented with L-glutamine or with control during the first month of life.**

| Brain structures (in cm³) | Control | Glutamine | Term infants | Effect size* | P-value* |
|---|---|---|---|---|---|
| Subcortical structures | 62.9 (5.81) | 66.5 (8.2) | 68.5 (4.9) | 0.50 | 0.06 |
| - Brain stem | 17.6 (2.2) | 18.9 (2.5) | 19.2 (1.8) | 0.54 | 0.04 |
| - Thalamus | 14.6 (1.5) | 15.3 (2.1) | 16.0 (1.1) | 0.39 | 0.14 |
| - Putamen | 10.1 (1.1) | 10.9 (1.6) | 10.9 (1.0) | 0.55 | 0.06 |
| - Caudate Nucleus | 7.3 (1.1) | 7.4 (1.4) | 8.0 (1.0) | 0.10 | 0.86 |
| - Hippocampus | 6.9 (0.6) | 7.2 (0.8) | 7.4 (0.8) | 0.47 | 0.04 |
| - Globus Pallidum | 3.3 (0.3) | 3.5 (0.4) | 3.5 (0.3) | 0.48 | 0.10 |
| - Amygdala | 2.2 (0.39) | 2.3 (0.4) | 2.3 (0.4) | 0.46 | 0.08 |
| - Nucleus accumbens | 1.0 (0.18) | 1.0 (0.3) | 1.1 (0.2) | 0.04 | 0.82 |
| Corpus callosum (in mm²) | 521.0 (72.0) | 535.7 (90.0) | 558.4 (80.7) | 0.18 | 0.40 |

| | | | | | |
|---|---|---|---|---|---|
| * For control versus glutamine group | | | | | |

The improved brain development established at school age is indicative for improvement in neurocognitive functioning, behavioral and/or motional development, improving neuronal activation during interference task or during visuo-spatial working memory task, improvement of visuomotor skills (caterpillar-task). In particular these data are indicative of improved neurocognitive functioning. fMRI (functional MRI data) indicate a neuronal activation more similar to term infants, in the glutamine fed preterm group later in life.

### Example 2: Effect of glutamine supplementation in VLBW and/or very preterm infants on brain volume later in life

Intellectual development was measured in the same group of infants as example 1 by a short form of the Wechsler Intelligence Scale for Children-III (WISC-III), including the subtests Vocabulary and Block Design. 68 infants completed the tests. Both subtests correlate strongly (r 0·90) with full-scale intelligence quotient (FSIQ). Scores on this test were normalised with a mean of 100 (SD 15). There was a trend (p=0.12 when adjusted for gestational age and socioeconomic status) in improved IQ (WISC-III FSIQ being 102, sd 17.7, n=30) in the glutamine group compared to the control group (WISC-III FSIQ 93.6 sd 17.2 n=34).

### Example 3: Preterm formula enriched in glutamine

Preterm formula in powder form comprising per 100 g about 486 kcal, 15.1 g protein, 46.1 g digestible carbohydrates, 26.7 g fat and 4.8 g non-digestible oligosaccharides. Furthermore the composition comprises minerals, trace elements, vitamins, L-carnitine, choline, myo-inositol and taurine as known in the art and according to guidelines for preterm infants. For a ready to drink form, about 16.5 g powder is reconstituted with water until a final volume of 100 ml.
The protein comprises about 85 wt.% casein and whey protein from cow's milk based on total protein in a weight ratio of 1:1.5. About 15 wt.% of the protein is free glutamine.
The non-digestible oligosaccharides are galacto-oligosaccharides (Vivinal GOS) and fructopolysaccharides (RaftilinHP) in a weight ratio of 9:1.

### Example 4: Nutritional supplement enriched in glutamine.

Human milk fortifier in powder form packed in sachets comprising about 2 g.
Based on 100 g powder the composition comprises 361 kcal, 19 g protein, 71.5 g digestible carbohydrates, the rest being minerals, trace elements and vitamins. The protein consists of free glutamine.

## Claims

1. Glutamine enriched nutritional composition for use in providing nutrition to a low birth weight and/or preterm infant for use in improving neurocognitive functioning at an age from 4 to 12 years.

2. The glutamine enriched nutritional composition for use according to claim 1 wherein the low birth weight or preterm infant is a very low birth weight infant or a very premature infant.

3. The glutamine enriched nutritional composition for use according to claim 1 or 2 wherein the daily dose of glutamine provided to the infant is at least 0.01 to 0.5 g/kg body weight, preferably 0.03 to 0.4, even more preferably 0.07 to 0.35 g/kg body weight.

4. The glutamine enriched nutritional composition for use according to any one of claims 1 to 3, wherein the nutritional composition is administered to the infant during the first month after birth.

5. The glutamine enriched nutritional composition for use according to any one of claims 1 to 4, wherein the nutritional composition is a preterm formula.

6. The glutamine enriched nutritional composition for use according to any one of claims 1 to 5, wherein the nutritional composition comprises between 1.5 wt.% and 20 wt.% glutamine based on dry weight of the nutritional composition, and comprises protein in such an amount that glutamine is present between 12 wt.% and 80 wt% based on total protein.

7. The glutamine enriched nutritional composition for use according to any one of claims 1 to 6, wherein the glutamine in the nutritional composition is in the form of free glutamine, glutamine dipeptide and/or glutamine tripeptide, preferably free glutamine and/or glutamine dipeptide.

8. The glutamine enriched nutritional composition for use according to any one of claims 1 to 7, wherein the nutritional composition further comprises non-digestible oligosaccharides selected from the group consisting of galacto-oligosaccharides and fructo-oligosaccharides.

9. The glutamine enriched nutritional composition for use according to claim 8, wherein the nutritional composition comprises at least 0.5 wt.% non digestible oligosaccharides based on dry weight of the composition.

10. The glutamine enriched nutritional composition for use according to any one of claims 8 or 9, wherein the nutritional composition comprises galacto-oligosaccharides.

11. The glutamine enriched nutritional composition for use according to any one of claims 1 to 10 wherein the nutritional composition is a glutamine-based supplement in the form of a powder in a unit dose comprising at least 15 wt.% glutamine in the form of free glutamine, glutamine dipeptide and/or glutamine tripeptide on dry weight of the powder.

12. The glutamine enriched nutritional composition for use according to claim 11 wherein the glutamine-based supplement comprises at least 10 wt.% non digestible oligosaccharides based on dry weight of the glutamine-based supplement.

## Patentansprüche

1. Glutamin-angereicherte Nahrungszusammensetzung zur Verwendung bei der Bereitstellung einer Nahrung für ein untergewichtig und/oder zu früh geborenes Kind zur Verwendung in der Verbesserung der neurokognitiven Tätigkeit im Alter von 4 bis 12 Jahren.

2. Die Glutamin-angereicherte Nahrungszusammensetzung zur Verwendung nach Anspruch 1, wobei das untergewichtig oder zu früh geborene Kind ein sehr untergewichtig oder sehr frühzeitig geborenes Kind ist.

3. Die Glutamin-angereicherte Nahrungszusammensetzung zur Verwendung nach Anspruch 1 oder 2, wobei die dem Kind verabreichte tägliche Glutamin-Dosis mindestens 0,01 bis 0,5 g/kg Körpergewicht, vorzugsweise 0,03 bis 0,4, und noch bevorzugter 0,07 bis 0,35 g/kg Körpergewicht beträgt.

4. Die Glutamin-angereicherte Nahrungszusammensetzung zur Verwendung nach einem der Ansprüche 1 bis 3, wobei die Nahrungszusammensetzung dem Kind während des ersten Monats nach der Geburt verabreicht wird.

5. Die Glutamin-angereicherte Nahrungszusammensetzung zur Verwendung nach einem der Ansprüche 1 bis 4, wobei die Nahrungszusammensetzung eine Formulierung für Frühgeborene ist.

6. Die Glutamin-angereicherte Nahrungszusammensetzung zur Verwendung nach einem der Ansprüche 1 bis 5, welche zwischen 1,5 Gew.-% und 20 Gew.-% Glutamin, bezogen auf das Trockengewicht der Nahrungszusammensetzung, und Protein in einem solchen Anteil umfasst, dass das Glutamin mit zwischen 12 Gew.-% und 80 Gew.-%, bezogen auf das Gesamtprotein, vorliegt.

7. Die Glutamin-angereicherte Nahrungszusammensetzung zur Verwendung nach einem der Ansprüche 1 bis 6, wobei das Glutamin in der Nahrungszusammensetzung in Form von freiem Glutamin, Glutamindipeptid und/oder Glutamintripeptid, vorzugsweise freiem Glutamin und/oder Glutamindipeptid vorliegt.

8. Die Glutamin-angereicherte Nahrungszusammensetzung zur Verwendung nach einem der Ansprüche 1 bis 7, welche weiterhin unverdauliche Oligosaccharide umfasst, die aus der Gruppe ausgewählt sind, die aus Galactooligosacchariden und Fructooligosacchariden besteht.

9. Die Glutamin-angereicherte Nahrungszusammensetzung zur Verwendung nach Anspruch 8, wobei die Nahrungszusammensetzung mindestens 0,5 Gew.-% unverdauliche Oligosaccharide, bezogen auf das Trockengewicht der Zusammensetzung, umfasst.

10. Die Glutamin-angereicherte Nahrungszusammensetzung zur Verwendung nach Anspruch 8 oder 9, wobei die Nahrungszusammensetzung Galactooligosaccharide umfasst.

11. Die Glutamin-angereicherte Nahrungszusammensetzung zur Verwendung nach einem der Ansprüche 1 bis 10, wobei die Nahrungszusammensetzung ein auf Glutamin basierendes Nahrungsergänzungsmittel in Form eines Pulvers mit einer Einheitsdosis ist, die mindestens 15 Gew.-% Glutamin, bezogen auf das Trockengewicht des Pulvers, in Form von freiem Glutamin, von Glutamindipeptid und/oder Glutamintripeptid umfasst.

12. Die Glutamin-angereicherte Nahrungszusammensetzung zur Verwendung nach Anspruch 11, wobei das auf Glutamin basierende Nahrungsergänzungsmittel mindestens 10 Gew.-% unverdauliche Oligosaccharide, bezogen auf das Trockengewicht des auf Glutamin basierenden Nahrungsergänzungsmittels, umfasst.

## Revendications

1. Composition nutritionnelle enrichie en glutamine destinée à être utilisée pour fournir un apport nutritionnel à un nourrisson à faible poids de naissance et/ou né avant terme afin d'améliorer son fonctionnement neurocognitif entre 4 et 12 ans.

2. Composition nutritionnelle enrichie en glutamine destinée à être utilisée selon la revendication 1, dans laquelle le nourrisson à faible poids de naissance ou né avant terme est un nourrisson à très faible poids de naissance ou un grand prématuré.

3. Composition nutritionnelle enrichie en glutamine destinée à être utilisée selon la revendication 1 ou 2, dans laquelle la dose journalière de glutamine fournie au nourrisson est comprise entre au moins 0,01 et 0,5 g/kg de son poids corporel, de préférence entre 0,03 et 0,4, de préférence encore entre 0,07 et 0,35 g/kg de son poids corporel.

4. Composition nutritionnelle enrichie en glutamine destinée à être utilisée selon l'une quelconque des revendications 1 à 3, dans laquelle la composition nutritionnelle est administrée au nourrisson au cours du premier mois suivant la naissance.

5. Composition nutritionnelle enrichie en glutamine destinée à être utilisée selon l'une quelconque des revendications 1 à 4, dans laquelle la composition nutritionnelle est une préparation pour prématurés.

6. Composition nutritionnelle enrichie en glutamine destinée à être utilisée selon l'une quelconque des revendications 1 à 5, dans laquelle la composition nutritionnelle comprend entre 1,5 % en poids et 20 % en poids de glutamine par rapport au poids sec de la composition nutritionnelle et comprend des protéines en quantité telle que la glutamine représente entre 12 % et 80 % du poids total de protéines.

7. Composition nutritionnelle enrichie en glutamine destinée à être utilisée selon l'une quelconque des revendications 1 à 6, dans laquelle la glutamine contenue dans la composition nutritionnelle se présente sous forme de glutamine libre, de dipeptide de glutamine et/ou de tripeptide de glutamine, de préférence de glutamine libre et/ou de dipeptide de glutamine.

8. Composition nutritionnelle enrichie en glutamine destinée à être utilisée selon l'une quelconque des revendications 1 à 7, dans laquelle la composition nutritionnelle comprend en outre des oligosaccharides non digestibles choisis dans le groupe comprenant les galacto-oligosaccharides et les fructo-oligosaccharides.

9. Composition nutritionnelle enrichie en glutamine destinée à être utilisée selon la revendications 8, dans laquelle la composition nutritionnelle comprend au moins 0,5 % en poids d'oligosaccharides non digestibles par rapport au poids sec de la composition.

10. Composition nutritionnelle enrichie en glutamine destinée à être utilisée selon l'une quelconque des revendications 8 ou 9, dans laquelle la composition nutritionnelle comprend des galacto-oligosaccharides.

11. Composition nutritionnelle enrichie en glutamine destinée à être utilisée selon l'une quelconque des revendications 1 à 10, dans laquelle la composition nutritionnelle est un complément alimentaire à base de glutamine sous forme de poudre en dose unitaire comprenant au moins 15 % en poids de glutamine sous forme de glutamine libre, de dipeptide de glutamine et/ou de tripeptide de glutamine par rapport au poids sec de la poudre.

12. Composition nutritionnelle enrichie en glutamine destinée à être utilisée selon la revendication 11, dans laquelle le complément alimentaire à base de glutamine comprend au moins 10 % en poids d'oligosaccharides non digestibles par rapport au poids sec du complément alimentaire à base de glutamine.
